(19) Europäisches Patentamt / European Patent Office / Office européen des brevets

(11) **EP 3 872 494 A1**

(12) **EUROPEAN PATENT APPLICATION**
published in accordance with Art. 153(4) EPC

(43) Date of publication:
**01.09.2021 Bulletin 2021/35**

(21) Application number: **19877162.8**

(22) Date of filing: **24.10.2019**

(51) Int Cl.:
***G01N 33/68*** (2006.01)

(86) International application number:
**PCT/KR2019/014027**

(87) International publication number:
**WO 2020/085803 (30.04.2020 Gazette 2020/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.10.2018 KR 20180127909**

(71) Applicant: **Seoul National University R & DB Foundation**
**Seoul 08826 (KR)**

(72) Inventors:
• **MOOK, Inhee**
**Seoul 04386 (KR)**
• **LEE, Sang-Won**
**Seoul 02843 (KR)**
• **HWANG, Daehee**
**Suwon-si Gyeonggi-do 16420 (KR)**
• **HAN, Sun-Ho**
**Seoul 06278 (KR)**
• **PARK, Jong-Chan**
**Seoul 05400 (KR)**

(74) Representative: **Ter Meer Steinmeister & Partner Patentanwälte mbB**
**Nymphenburger Straße 4**
**80335 München (DE)**

(54) **BLOOD BIOMARKER FOR DETECTING DEPOSITION OF AMYLOID BETA IN BRAINS OF GROUPS WITH NORMAL COGNITIVE FUNCTION AND MILD COGNITIVE IMPAIRMENT**

(57) Disclosed in the present application is a biomarker capable of early diagnosing amyloid beta deposition in the brain and a disease associated therewith through a blood test. The marker according to the present application can make use of blood to detect the deposition of amyloid beta in brains of the groups with normal cognitive function and mild cognitive impairment before the appearance of a clinical symptom and as such, can be conveniently and advantageously used to early diagnose a disease associated therewith.

**FIG. 1**

**Description**

**BACKGROUND OF INVENTION**

**Field of the Invention**

The present disclosure generally relates to predicting the accumulation of amyloid beta in the brain of subjects with normal cognitive function or mild cognitive impairment using blood test, and to early diagnosis of disease associated therewith.

**Description of the Related Art**

Alzheimer's disease (AD), a representative disease caused by accumulation of beta amyloid in the brain, is the most common form of dementia and is a representative neurodegenerative disease. It is estimated that more than 20% of the elderly over the age of 80 are affected by Alzheimer's disease, and the number is increasing rapidly as the society becomes aged. In Alzheimer's disease, the major pathological characteristics is the senile plaques caused by the accumulation of β-amyloids (Aβ peptide) in the brain tissues, which are produced by the sequential cleavage of amyloid precursor protein (APP) by β, and γ-secretase, and the neurofibrillary tangle (NTF) caused by hyperphosphorylation of the Tau proteins, a microtubule-associated protein.

Currently, the most common AD diagnosis methods include imaging methods such as magnetic resonance imaging (MRI) and positron emission tomography (PET), and indirect methods such as mini mental state examination (MMSE) and medical examination are also used. However, diagnosis through MMSE gives different results depending on age, education, etc., so the accuracy of diagnosis is a problem. Also used is a method of identifying two lesions in the brain tissue of AD patients, the senile plaque and the nerve fiber mass. However, no methods are known for diagnosing the disease or reliably determining the degree of progress. Research to find biochemical diagnostic markers for AD diagnosis is an important research field and is being conducted using various tissues including body fluids such as blood and cerebrospinal fluid and the like. Cerebrospinal fluid (CSF) is a site in direct contact with the outer area of the cells of the brain and reflects changes in the proteins of the brain. Compared to the normal elderly, it has been reported that the Aβ42 concentration is decreased and the total concentrations of tau and autophosphorylated tau are increased in the CSF of Alzheimer's disease patients. However, considering that most AD patients are aged 65 years or older, the use of lumber puncture to obtain cerebrospinal fluid may cause a significant risk to the patient.

Pittsburgh compound B (PiB), recently developed as a causative agent of Alzheimer's dementia and specifically binding to amyloid beta, which accumulates in the brain as the disease progresses, is a useful substance for the diagnosis of Alzheimer's dementia. This is because the level of accumulation of amyloid beta in the brain can be measured by taking PET images after administration of PiB, and this can be effectively used for diagnosis of Alzheimer's dementia. However, this diagnostic method has limitations in its use as a general diagnosis method for Alzheimer's dementia due to its high cost and the limited availability.

Compared to this, considering the relative ease of obtaining samples, low cost, and time savings, using blood diagnostic markers have many advantages. Blood diagnostic markers should be able to reflect functional and pathological changes in the brain as Alzheimer's disease progresses, However, changes in the various brain proteins according to Alzheimer's disease progression are not always detected in blood, making it difficult to find blood diagnostic markers. In addition, it is a generalized phenomenon that the brain proteins that are increased or decreased according to the progression of Alzheimer's disease show the opposite tendency to increase or decrease in cerebrospinal fluid or blood. For example, as Alzheimer's disease progresses, the concentration of amyloid beta 42 (Aβ 42) increases in the brain while it decreases in the cerebrospinal fluid. Further the concentration of transthyretin increases in the brain while it decreases in the blood (Scheuner et al., Nature Medicine 2, 864-870 (1996); Galasko et al., Arch Neurol 55(7):937-45 (1998); Li et al., Journal of Neurosci ence 31(35):12483-12490 (2011); and Han et al., Journal of Alzheimer disease 25(1)77-84 (2011)).

Korean Patent Application Publication No. 2012-0041823 relates to a protein marker for early diagnosis of Alzheimer's disease, and discloses a marker for early diagnosis of Alzheimer's disease using ATP synthase subunit beta and adenosine kinase (Isoform Long) and regucalcin.

Korean Patent Application Publication No. 2010-0049363 relates to a diagnosis apparatus and method for diagnosis of Alzheimer's disease using a vitamin D-binding protein, and discloses a diagnosis method and apparatus using an antibody that binds to a vitamin D-binding protein.

Korean Patent Application Publication No. 2014-0042331 relates to a multi-marker for diagnosing cognitive disorders and their uses. As diagnostic markers for cognitive disorders, transthyretin, ApoE, alpha-synuclein, and vitamin D binding protein, neurorogranin, vimentin, stathmin, contactin, and HDL-cholesterol are disclosed.

However, considering that Alzheimer's disease is difficult to diagnose early and that it is a serious disease that makes the patient unable to live a normal life thus requiring a lot of social cost if the disease continues to progress, there is a need to develop new markers that can be used for early diagnosis of Alzheimer's disease through a simple sample such as blood, especially in the preclinical stage before symptoms appear.

Recently, various blood biomarker candidates for the diagnosis of Alzheimer's dementia have been developed through extensive research, but such biomarker does not meet the required criteria in terms of sensitivity and accuracy.

## SUMMARY OF THE INVENTION

The present disclosure is to develop a biomarker capable of predicting the accumulation of beta amyloid in the brain or early diagnosis of a disease related thereto by using a blood sample.

In one aspect, the present disclosure provides a use of Galectin-3 as a blood biomarker for detecting the accumulation of amyloid beta in the brain in particular of the subject belong to a non-dementia group having a normal cognitive function and mild cognitive impairment, and the use of Galectin-3 for early diagnosis of the disease associated with the accumulation of amyloid beta in the brain.

In one embodiment of the present disclosure, there is provided a composition for use in detection of the accumulation of amyloid beta in the brain of a subject having a normal cognitive function or mild cognitive impairment, comprising an agent for detecting Galectin-3 as a biomarker in the blood of the subject, wherein the agent is for quantifying Galectin-3 amount.

The concentration/amount/level of the present biomarker Galectin-3 in blood is increased in the group having brain amyloid accumulation with a normal cognitive function or mild cognitive impairment.

The present biomarker may further comprise at least one of ACE (angiotensin converting enzyme), LGALS3BP, and ApoE for use as a combination for detecting the accumulation of amyloid beta in the brain.

The present biomarker may further comprise beta-amyloid 1-42 or a ratio of 1-42/1-40 in the blood for use as a combination and the blood level of beta-amyloid 1-42 or the ratio of 1-42/1-40 is decreased in the blood of the subject having brain amyloid accumulation with a normal cognitive function or mild cognitive impairment.

The present biomarker for detecting the brain amyloid beta accumulation is also used for early diagnosis of diseases related to brain amyloid beta accumulation, or for determining whether a Pittsburgh compound B (PIB)-positron emission tomography (PET) test is necessary for the subject, wherein the disease related to the brain amyloid beta accumulation disease includes Alzheimer's disease, Parkinson's disease dementia, Lewy body dementia, Huntington's disease dementia, or pre-clinical Alzheimer's disease, Down syndrome, or cognitive impairment.

In other aspect, the present disclosure provides a method of detecting a brain amyloid accumulation of a subject in need of thereof, comprising the steps of: providing a blood sample from the subject; quantifying Galectin-3 biomarker in the blood sample; and correlating the quantified result or the amount of the biomarker with a brain amyloid beta accumulation in the subject; wherein the subject belongs to a cognitively normal group or mild cognitive impairment group.

In one embodiment, the present methods further comprise in the correlation step determining that the subject from whom the blood sample is derived has a brain amyloid beta accumulation when the amount of the Galectin-3 biomarker is increased compared to the amount determined in the control group.

In other embodiment, the present method further comprises as biomarkers at least one of LGALS3BP, ACE (Angiotensin Converting Enzyme), or ApoE, in which case, the correlation step further comprises determining that the subject has a brain amyloid beta accumulation when the amount of LGALS3BP is decreased, the amount of ACE is decreased in comparison to the amount determined in the control group, or the subject has a ApoE 4 allele.

In other embodiment, the present method further comprises as biomarkers a beta-amyloid 1-42 or a ratio of 1-42/1-40 in blood, in which case the correlation step further comprises determining that the subject has a brain amyloid beta accumulation when the amount of the beta-amyloid 1-42 or the ratio of 1-42/1-40 in the blood is decreased in comparison to those of the biomarkers determined in the control group.

The present blood biomarker Galectin-3 allows for predicting or detecting the accumulation of amyloid beta in the brain which is known as a causative agent of Alzheimer's dementia, and thus for early diagnosis of Alzheimer's dementia before symptoms appear. Such detection and diagnosis using a blood sample has a superior advantage compared to the existing method of detecting the accumulation of amyloid beta in the brain in terms of cost and convenience.

## BRIEF DESCRIPTION OF THE DRAWINGS

These and/or other aspects and advantages of the invention will become apparent and more readily appreciated from the following description of the embodiments, taken in conjunction with the accompanying drawings of which:

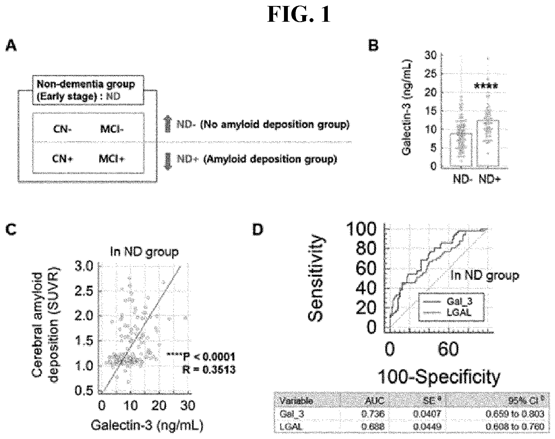

Fig 1 is the results of showing that there is significant relationship between the concentration of galectin-3 in the blood and the concentration of amyloid beta in the brain of cognitively normal and mild cognitive impairment group (non-dementia group). Fig. 1, A shows the characteristics of the group used in the experiment of the present application. The group with brain accumulation of amyloid beta in the non-dementia group (ND) including the cognitively normal (CN) and the mild cognitive impairment (MCI) group is indicated as ND+ and the group without the accumulation is denoted as ND-. Fig. 1, B is the results of analyzing the quantification of galectin-3 by ELISA in the plasma of ND- and ND+ group. It shows a significant increase in the concentration of Galectin-3 in ND+ compared to ND-group (****p <0.0001). (unpaired t-test). In Fig. 1, C, using partial correlation analysis, it was confirmed that there is a significant correlation between the plasma galectin-3 concentration and the degree of amyloid accumulation in the brain (p<0.0001, r=0.3513). Fig.1, D shows that the AUC value of Galectin-3 significantly increased from 0.688 to 0.736 compared to the existing marker (LGALS3BP) determined by ROC curve analysis that analyzes the specificity and sensitivity of biomarkers, which indicates the present biomarker Galectin-3 is superior in specificity and sensitivity compared to the existing marker.

Fig. 2 shows the change in the significance of the ROC curve when the concentration of galectin-3 was added to the analysis compared to when the concentration ratio of beta-amyloid in the blood was used alone.

Fig. 3 shows that the AUC value is improved from 0.792 to 0.856 when Galectin-3 was added to the existing biomarker combination (LGALS3BP, ACE).

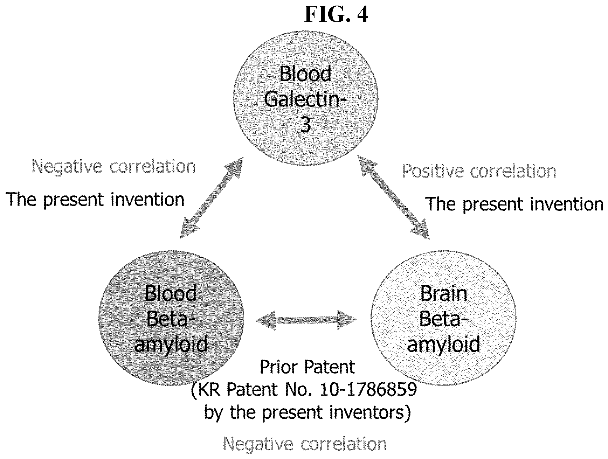

Fig. 4 shows schematically the summary of the experimental results of the present invention.

## DETAILED DESCRIPTION OF THE EMBODIMENTS

The present invention is based on the discovery that the blood concentration of Galectin-3 is closely related to the accumulation of amyloid beta in the brain, and thus it can be used as a blood biomarker for early diagnosis of diseases related thereto and for determination of the accumulation of amyloid beta in the brain.

Accordingly, in one aspect, the present disclosure relates to a Galectin-3 biomarker capable of detecting or predicting amyloid beta accumulation in the brain using blood sample and its use, particularly in the cognitively normal and mild cognitive impairment group, both of which are classified as non-dementia group.

The biomarker according to the present disclosure may be implemented in the form of a composition containing detecting agents that can detect the present biomarker *in vitro* or *in vivo,* or in the form of a kit, or in the form of a composition containing the marker itself, or of a method for detecting/determining by detecting the present biomarker in the blood.

In one embodiment, the present disclosure relates to a composition for use in detecting the accumulation of amyloid beta in the brain by detection of Galectin-3 biomarker in the blood, in which the composition comprises a substance capable of detecting Galectin-3, and the blood is from a cognitively normal group or mild cognitive impairment group.

Galectin-3 is a protein encoded by a gene called LGALS3. The human protein sequence Galectin-3 may be found, for example as the UniProt ID P17931. In the present disclosure, the present marker can be used to test the accumulation of amyloid beta in the brain by blood tests particularly in cognitively normal group or mild cognitive impairment group, both of which are classified as non-dementia group. The concentration of Galectin-3 is increased in the blood from cognitively normal and mild cognitive impairment group with the accumulation of beta amyloid in the brain.

Amyloid beta is known as a causative agent of Alzheimer's dementia (AD) and is a representative pathogen of AD that accumulates in the brain as the disease progresses. Pittsburgh compound B (PIB), recently developed as a compound that specifically binds to beta amyloid, is a useful agent in the diagnosis of Alzheimer's dementia. This is because the level of accumulation of amyloid beta in the brain can be measured by taking a PET image after administration of PIB, which then can be used to diagnose Alzheimer's dementia. However, this diagnostic method has limitations in its use as a general diagnosis method for Alzheimer's dementia due to its high cost and limited equipment.

The accumulation of cerebral amyloid beta usually begins 15 to 20 years before any clinical symptoms such as dementia or forgetfulness appear, so if it is possible to confirm whether or not amyloid beta is accumulated in the brain through by simple blood tests in the patients with no clinical symptoms or showing mild clinical symptoms, Alzheimer's dementia can be diagnosed early and thus the progression of the disease can be slow down or prevented. Therefore, the blood biomarker according to the present disclosure that can determine/predict the accumulation of amyloid beta in the brain is of great importance.

In addition, the detection of the accumulation of beta amyloid in the brain using the present biomarker can also be used for diagnosis of diseases caused by accumulation of beta amyloid in the brain.

Specifically, various diseases caused by the accumulation of beta amyloid in the brain are known (Head, E., and Lott, I. T. (2004) Down syndrome and beta-amyloid deposition. Curr Opin Neurol 17; Primavera et al., (1999) Brain Accumulation of Amyloid-beta in Non-Alzheimer Neurodegeneration. J Alzheimers Dis; Masliah et al., (2001) beta-amyloid peptides enhance alpha-synuclein accumulation and neuronal deficits in a transgenic mouse model linking Alzheimer's disease and Parkinson's disease. Proc Natl Acad Sci). Therefore, the marker according to the present disclosure can also be used for diagnosis, detection, etc. of various diseases related to the brain amyloid beta accumulation.

In one embodiment, the disease associated with such brain amyloid beta accumulation includes, for example, Alzheimer's disease, Parkinson's disease dementia, Lewy body dementia, Huntington's disease dementia, or preclinical Alzheimer's disease, Down syndrome, or cognitive impairment.

As used herein, "cognitive impairment" refers to a neurodegenerative disease, for example, includes Alzheimer's disease (AD) dementia, Parkinson's disease dementia, Lewy body dementia or Huntington's disease dementia, patients having Alzheimer's dementia (ADD), and mild cognitive impairment (MCI), which is a stage before the progression into dementia and in this context includes dementia and non-dementia group of patients. The severity of such cognitive impairment can be determined by methods such as MMSE (Mini mental state examination, 2006_Benson et al., Journal of clinical Psychiatry, 2008_O'Bryant et al., Arch Neurol) score.

As used herein, "dementia" includes Alzheimer's disease, Parkinson's disease dementia, Lewy body dementia, or Huntington's disease dementia.

As used herein, "non-dementia" includes a cognitively normal group and a mild cognitive impairment group.

As used herein, "cognitively normal group" refers to a group whose cognitive function is determined to be normal through the above-described examination such as MMSE, regardless of whether or not beta amyloid is accumulated in the brain. It includes people whose cognitive function is normal but with amyloid beta accumulated in the brain. When beta amyloid is accumulated in the brain, it may progress to dementia through mild cognitive impairment, so it is very important to determine whether or not beta amyloid is accumulated in the brain in the cognitively normal group for early detection of dementia.

As used herein, "mild cognitive impairment group" is a pre-stage of dementia that has a decline in cognitive function, but is capable of doing daily life, and includes a patient with or without beta-accumulation of amyloid in the brain. do. Determining whether or not beta amyloid is accumulated in the brain is crucial for early detection of dementia.

As used herein, "diagnosis" refers to determining the susceptibility of a subject to a specific disease or disorder, determining whether a subject currently has a specific disease or disorder, or determining the prognosis of an affected subject or therametrics (e.g, monitoring the condition of a subject to provide information about treatment efficacy).

Early diagnosis herein includes diagnosis at the pre-clinical stage including before the onset of clinical symptoms, normally cognitive or mild cognitive impairment stage,

In addition, if necessary, the present biomarker may be used in parallel or in combination with the existing blood markers for amyloid beta accumulation in the brain for accurate early diagnosis and prevention of Alzheimer's dementia. The present biomarker has an advantage of being able to be used in combination with such a biomarker that has already been discovered or will be discovered in the future, so that the utilization may be further increased.

In this context, the composition or method according to the present disclosure further comprises at least one marker selected from the group consisting of angiotensin converting enzyme (ACE), LGALS3BP, and ApoE. ACE and LGALS3BP are known as Uniprot ID Q08380 and P12821, respectively for their protein and nucleic acid sequences, intracellular processing, and modifications and the like, and thus those skilled in the art will be able to easily select a sequence and/or a detection method required for detection of the markers. When the markers are compared with the quantitative result determined in the control group, the LGALS3BP blood concentration decreases and the ACE blood concentration decreases when amyloid beta is accumulated in the brain.

In another embodiment, the composition or method according to the present disclosure may further comprise a use of ApoE marker. There are three allele types of ApoE gene: ApoE 2, ApoE 3, and ApoE 4. ApoE 4 is a very high-risk factor in which one with ApoE 4 has a high-risk of developing amyloid accumulation in the brain and AD compared one with other types of ApoE allele. The present biomarker may be added to the use of ApoE marker to improve its specificity and sensitivity. Detection of the ApoE genotype can be performed using known methods, and those skilled in the art will be able to select an appropriate one. For example, a PCR method using allele-specific primers can be used.

In another embodiment, the present biomarker further comprises a use of blood beta-amyloid 1-42 or a ratio of 1-42/1-40, and the beta-amyloid 1-42 or ratio of 1-42/1-40 is decreased in the blood from the cognitively normal group and mild cognitive impairment group with amyloid beta accumulated in the brain.

As used herein, "amyloid beta plaque" or "beta amyloid plaque" is an insoluble fibrous protein aggregate containing amyloid beta, also referred to herein as A-beta, and the main components of which is A-beta 40 and A-beta 42. In one embodiment, the amyloid plaque may be present in a cell, on a surface of a cell, and/or in a space between cells. In particular, it exists in the space between the cells of the neural tissue and is used as a marker for the diagnosis of Alzheimer's dementia, and the diagnosis of dementia according to the degree of plaque accumulation can be referred to as disclosed (Mawuenyega et al., Science, 2010_Querfurth and LaFerla, The New England journal of medicine). The amyloid beta plaque may be referred to amyloid beta depending on the context, which will be readily determined by a person skilled in the art.

As used herein, the term "biological sample or specimen" includes, but is not limited to, any solid or liquid sample obtained from a human body or a mammal, such as a tissue derived from a specific organ, urine, saliva, whole blood, platelets, plasma or serum samples.

The markers according to the present disclosure is in particular detected in blood samples. The blood sample can be obtained very easily compared to other samples such as cerebrospinal fluid used for diagnosis of dementia, and thus the convenience is greatly increased as well as the cost reduction. Samples for which the markers according to the present disclosure are used include, but are not limited to, whole blood, platelets, plasma or serum samples. In one embodiment, plasma is used. As the samples employed herein, a sample from a test subject in need of diagnosis or detection, as well as a sample from a normal control group or a control group having a specific disease for comparative analysis, may also be used.

In the present disclosure, plasma amyloid beta is treated with a plasma pretreatment composition (MPP) which is a mixture of protease inhibitors and phosphatase inhibitors. For this, reference may be made to what is disclosed in detail in Korean Patent Application Publication No. 2016-0129444 filed by the present inventor.

As used herein, "detection reagent or agent" is a reagent or substance capable of detecting or quantifying a marker according to the present disclosure, for example, a substance capable of detecting the markers of the present disclosure at a nucleic acid level such as gene or mRNA and/or at a protein level and also the reference may be made to those described in the present Examples.

As used herein, detection includes quantitative and/or qualitative analysis, and includes detection of the presence or absence, or detection of the expression levels, and such methods are known in the art. Thus those skilled in the art will be able to select a method suitable for the practice of the present invention in consideration of the description of the present disclosure including the following Examples. For example, the detection of the marker Galectin-3 may be referred to, for example, the methods described in the Examples herein.

For example, methods and reagents for detection of the markers at the protein level are known. For example, antigen-antibody reactions, substrates that specifically bind to the markers, receptors or ligands or cofactors that specifically interact with the markers may be used. The reagent or material that specifically interacts or binds to the marker of the present disclosure may be used in a chip method or in combination with nanoparticles. In one embodiment, the antigen-antibody reaction is carried out by enzyme linked immunosorbent assay (ELISA), radioimmunoassay (RIA), sandwich ELISA, western blot on polyacrylamide gel, immune dot blot. Immuno dot blotting assay, Immuno-fluorescence Assay (IFA), Immunochemiluminescence Assay, Immunohistochemical staining or immunochromatography(Rapid), antigen-antibody reaction using beads or disc (X-MAP technology) and the like.

According to one embodiment of the present disclosure, the composition according to the present disclosure includes reagents necessary for detection of a marker at the protein level. For example, reagents that can be used for detection at the protein level may include monoclonal antibodies, polyclonal antibodies, substrates, aptamers, receptors, ligands or cofactors. If necessary, these reagents may be used by being incorporated into nanoparticles or chips.

According to another embodiment of the present disclosure, the detection reagent includes an antibody, and the detection of the present markers is performed using an antibody molecule that specifically binds thereto.

Antibodies that can be used herein are polyclonal or monoclonal antibodies, preferably monoclonal antibodies. Antibodies may be prepared by methods commonly practiced in the art, for example, fusion methods (Kohler and Milstein, European Journal of Immunology, 6:511-519 (1976)), recombinant DNA methods (US Pat. No. 4,816,56). Alternatively, it can be prepared by the phage antibody library method (Clackson et al, Nature, 352:624-628 (1991) and Marks et al, J. Mol. Biol., 222:58, 1-597 (1991)). General procedures for antibody production are described in Harlow, E. and Lane, D., Using Antibodies: A Laboratory Manual, Cold Spring Harbor Press, New York, 1999; Zola, H., Monoclonal Antibodies: A Manual of Techniques, CRC Press, Inc., Boca Raton, Florida, 1984; and Coligan, CURRENT PROTOCOLS IN IMMUNOLOGY, Wiley/Greene, NY, 1991, which documents are incorporated herein by reference.

Accordingly, in another aspect, the marker or combination of the markers according to the present disclosure as described above may be used to determine the amyloid beta plaque accumulation, early diagnosis, or to determine whether PiB-PET imaging test is necessary. For example, if it is determined that amyloid beta is not accumulated in the brain of a subject in the blood test using the present marker, it is not necessary to test the PiB-PET image, and if it is determined that amyloid beta is accumulated in the brain of a subject in the blood test using the present marker, the PiB-PET image may be used for the confirmation.

In the present disclosure, we showed that the accumulation of amyloid beta plaques known as the causative agent of Alzheimer's dementia based on the brain imaging results of PiB-PET, which is widely used for the diagnosis of Alzheimer's dementia, is highly correlated with the concentration of the present blood biomarker. That is, in the PiB-PET positive subjects, compared with the results of the PiB-PET negative subjects, the concentration of the marker according to the present application in the blood is significantly increased. That is, referring to FIG. 1, the marker according to the present application is increased in PiB-PET protons. This indicates that the marker according to the present application can be effectively used for determining the accumulation of beta amyloid in the brain using blood sample.

The method of the present disclosure may additionally use non-protein clinical information of a patient, that is, clinical information other than the marker, in addition to the result of the marker analysis. Such non-protein clinical information includes, for example, but is not limited to, the patient's age, sex, weight, diet, body mass, underlying disease, magnetic resonance imaging (MRI), single-photon emission computed tomography (SPECT), or mini-mental status examination (MMSE) or positron emission tomography (PET).

The present method includes the step of correlating the detection/quantification result of the marker with the accumulation of amyloid beta plaque in the brain or the diagnosis or prognosis of a disease related thereto, and according to one embodiment, the step compares the determined amount of marker of the present disclosure with those from a control sample.

In one embodiment, as a control, normal sample including a sample from a person tested as negative in the PIB-PET may be used. And the value determined from a test subject is compared to the value from such control as to the increase or decrease for the prediction or diagnosis.

For example, when the marker according to the present disclosure is significantly increased compared to the value determined from the control group, the accumulation of plaque in the subject or the information for diagnosing the disease may be provided.

For example, in the correlation step, the concentration of the present marker in the sample of a subject with beta amyloid accumulation is increased as compared to that from the control, in which the control is a sample from a normal subject or from a subject tested negative in the PIB-PET test.

For example, when more than one marker is used at the same time (multi-marker) and the degree of change such as the increase or decrease is calculated as a single value, the accumulation of amyloid beta in the brain may be predicted if the value is above or below a certain cutoff.

In addition, in the correlation step according to one embodiment of the present disclosure, by analyzing a sample of a normal control and a subject in need of detection, a threshold for determining or predicting the accumulation of plaque or diagnosing the onset of the disease associated with the plaque for the present marker may be determined and the detection result can be compared with the threshold value. For example, the threshold for the marker that determines whether or not brain amyloid beta plaques are accumulated, that is, negative or positive, that is, PiB-PET negative and positive, may be determined based on YOUDEN index determined by ROC curve analysis using the Med Calculate program. The value above the threshold indicates positive for the accumulation of amyloid beta plaques in the brain, and may be used for selection of the subjects who needs PiB-PET testing, for determination of the accumulation of brain amyloid beta plaques, or diagnosis of related diseases associated the accumulation of brain amyloid beta plaques.

Since the PIB-PET test is not only inconvenient to the patient, but also requires a high cost and time, a screening process to determine the person with a high possibility of amyloid plaque accumulation is urgently needed.

In another aspect, the present disclosure relates to the methods of detecting the present marker to screen for the patients who requires a PIB-PET test in which the patients are a subject in need of the determination of the beta amyloid accumulation in the brain. In one embodiment the methods comprise the steps of providing a blood sample from the subject in need of such detection; quantifying the marker according to the present disclosure in the blood sample; and determining the subject requires a PIB-PET test by comparing the concentration of the marker of the subject with that of a control in which the control sample is the one found negative in PIB-PET test. For example, if it is determined that amyloid beta is not accumulated in the brain in the blood test using the marker according to the present disclosure, it is not necessary to test the PiB-PET, or if it is determined that amyloid beta is accumulated in the brain in the blood test using the marker according to the present disclosure, it may be necessary to test the PiB-PET for the confirmation.

In addition, in all the methods according to the present application described above, reference may be made to the above for the markers. The present disclosure is further explained in more detail with reference to the following examples. These examples, however, should not be interpreted as limiting the scope of the present invention in any manner.

## EXAMPLES

### Materials and Methods

### Blood Sampling

The patient group was recruited for the Korean Brain Aging Study for Early Diagnosis and Prediction of Alzheimer's Disease (KBASE). Blood was collected in the morning on K2 EDTA tubes (BD Vacutainer System, Plymouth, UK), and centrifuged at 700Xg for 5 minutes to obtain the supernatant plasma sample. The obtained plasma samples were divided and stored in a -80 °C cryogenic freezer until use.

### Pittsburgh compound B positron emission tomography; PiB-PET

PiB-PET imaging was performed using a 3.0 T PET-MR scanner (Biograp h mMR scanner [Siemens, Washington DC, USA]), and the imaging technique was based on a previously reported paper [Han, S.H., et al., Neurobiol Aging, 2019. 73: p. 21-29.]. Participants were divided into PiB+ (beta-amyloid-bearing group) and PiB- (beta-amyloid-free group) based on the standardized uptake value ratio (SUVR), and the participants were classified as PiB+ if the value is greater than 1.4 in at least one of the four ROIs and as PiB- if the value less than or equal to 1.4 [Reiman, E.M., et al., Proc Natl Acad Sci U S A, 2009. 106(16): p. 6820-5; Choe, Y.M., et al.. Neurobiol Aging, 2014. 35(7): p. 1519-25]. 18F-AV-1451 PET scan was performed for 10 minutes using a Biograph True point 40 PET/CT scanner (Siemens, Washington, DC, USA), and the imaging technique was based on a previously reported paper [Park, J.C., et al., Brain, 2019. 142(3): p. 771-786].

**Participants**

The experiment had 197 participants. The participants are divided into: cognitively normal/PiB-PET positive; CN-, n=79; cognitively normal/PiB-PET negative; CN+, n=27; mild cognitive impairment/PiB-PET negative; MCI-, n=21; mild cognitive impairment/PiB-PET+; MCI+, n=30; clinically dementia/PiB-PET+; Dementia+, n=40. The psychological or clinical evaluation, and blood tests and brain imaging tests (MRI and PiB-PET) were performed on all the participants. In addition, as a cognitive function test, the values from Mini-Mental State Examination (MMSE) test were used, and the MMSE z score corrected for the gender, age, and educational environment of the patients was finally used.

**Enzyme-Linked Immunosorbent Assay; ELISA**

The concentration of the protein galectin-3 in the blood was measured using an ELISA method. This is an experimental method to measure the amount of a specific protein in a biological sample using antigen-antibody reaction and colometric analysis. The experiments were done according to the manufacturer's instruction (Human Galectin-3 Quantikine ELISA kit DGAL30; R&D Systems). Briefly, each of the plasma or serum samples and the standard samples for which the concentrations are known were added to the wells coated with the antibody, and incubated in an incubator at 37° C for 2 hours. Then, after washing three times with a washing solution, biotin antibody was added and incubated again in an incubator at 37°C for 1 hour. After washing with the washing solution five times, the horseradish peroxidase (HRP)-avidin solution was used to bind the biotin antibody and incubated again in an incubator at 37° C. for 1 hour. Finally, after adding the TMB solution and incubating at room temperature for 5-10 minutes, the protein concentration was determined by reading the plate at 450 nm wavelength within 5 minutes.

**Measurement of the blood beta amyloid concentration**

The concentration of beta-amyloid in blood was measured using the X-MAP technique after treating the sample with the plasma pretreatment composition. The plasma pretreatment composition contains the protease inhibitor and the phosphatase inhibitor (MPP) at a ratio of 1:1 (v/v). In addition, MPP may be a mixture of a protease inhibitor cocktail (PIC): PMSF (Serine protease inhibitor): phosphatase inhibitor cocktail I: phosphatase inhibitor cocktail II at 1:1:1:1 (v/v). This is described in detail in the present inventor's patent [Korean Patent No. 1786859 on a method for clinically and pathologically monitoring Alzheimer's disease through the concentration of amyloid beta in plasma]. Briefly, in order to measure the concentrations of beta-amyloid 1-42 and 1-40 in the blood at the same time, an INNO-BIA plasma Aβ forms kit (Fujirebio) was used. The beads to which the antibody is bound were first passed through the plate, and the beads were mounted on the plate, and then the plasma solution sample treated with MPP was incubated for 30 minutes, and then put into the plate together with the standard (standard concentration) samples. Afterwards, beads bound with conjugate antibody were added and stored in the refrigerator for one day. The next day, after washing the plate, detection antibody was added and incubated for 1 hour. As a final step, the reading solution was added and the beta-amyloid pretreated with MPP in plasma was measured using X-MAP technology.

**Statistical Analysis**

The measured blood galectin-3 concentration was used as a biomarker variable using appropriate statistical analysis. Statistical analysis was performed using Graphpad Prism 7 (GraphPad Software, Sandiego, CA, USA) and MedCalc (MedCalc Software, Ostend, Belgium) software. All data were expressed as mean $\pm$ standard error of the mean (SEM). In addition, the PiB-PET positive or negative groups was compared through the plasma protein concentration using a single Student's t test. After ANOVA analysis, a Tukey's multiple comparison test was performed with GraphPad Prism 7. In order to evaluate the performance of the diagnostic test, logistic regression was performed according to Reveiver Operating Characteristic (ROC) curve analysis with MedCalc. In addition, using this data, an ROC curve was drawn and AUC (Area under the curve) was measured using Wilcoxon statistics. In multiple logistic regression analysis, each variable was used as an independent variable to derive a final model, in which the covariates such as gender and age were used as correction variables.

$$\ln\left(\frac{Pi}{1-Pi}\right) = \beta_0 + \beta_1 x_{1,i} + \beta_2 x_{2,i} +$$

$$\cdots + \beta_m x_{m,i}$$

[Logistic regression formula. Pi = probability, $\beta$ = constant, x = coefficient. For example, $x_1$ = blood galectin-3 concentration, $x_2$ = blood LGAL3SBP concentration...]

In addition, a new comprehensive variable derived from the logistic regression analysis was used to determine whether or not brain amyloid was accumulated through ROC curve analysis, and the cut-off value (Criterion value) obtained through regression analysis was used as a reference point for distinguishing PiB-PET positive and PiB-PET negative.

**EXAMPLE 1. Analysis of the relationship between blood Galectin-3 concentration and beta-amyloid accumulation in the brain**

In the present disclosure, it was found that the blood concentration of galectin-3 is significantly changed with the beta-amyloid accumulation in the brain in the mild cognitive impairment group and the cognitively normal group classified as non-dementia group. Specifically, in order to analyze the change in blood galectin-3 concentration in non-dementia group (ND), which combined all patients with cognitively normal and mild cognitive impairment, the ND group was divided into subgroups with (ND+) and without (ND-) beta-amyloid accumulated in the brain. When the concentration of galectin-3 was compared, it was confirmed that the concentration was increased in the ND+ group (see B of Fig. 1 and Table 1).

In addition, when the correlation analysis was performed to see the relationship between the continuous variables, it was confirmed that the protein concentration of galectin-3 in the blood of each patient and the concentration of beta-amyloid in the brain of each patient showed a significant correlation (C of Fig. 1, p<0.0001). In addition, when the ROC curve analysis was performed for galectin-3 and LGALS3BP, respectively, the AUC of LGALS3BP was 0.688, whereas the AUC of galectin-3 was highly improved as 0.736, (D of Fig. 1, Table 2). This indicates that when the present biomarker galectin-3 is used as a variable, the accumulation of beta-amyloid in the brain, a causative agent of Alzheimer's disease, can be predicted much better than that of the existing LGALS3BP biomarker.

[Table 1]

| Characteristics (n) | CN- (79) | CN+ (28) | MCI- (53) | MCI+ (54) |
|---|---|---|---|---|
| Gender, M/F | 24/45 | 15/13 | 16/37 | 20/34 |
| Age, years, mean ± SEM | 66.90 ± 0.9 | 74.89 ± 1.2 | 74.00 ± 1.0 | 73.91 ± 0.9 |
| Education, mean ± SEM | 11.82 ± 0.6 | 12.71 ± 0.8 | 8.81 ± 0.6 | 10.53 ± 0.6 |
| MMSE raw score, mean ± SEM | 27.05 ± 0.3 | 27.04 ± 0.4 | 22.89 ± 0.4 | 21.67 ± 0.4 |
| MMSE z score, mean ± SEM | 0.28 ± 0.1 | 0.31 ± 0.2 | -0.83 ± 0.2 | -1.57 ± 0.2 |
| CDR (n) | 0 | 0 | 0.5 (53) | 0.5 (50) |
| ApoE4 positivity, ε4+/N (%) | 13/79 (16%) | 10/28 (36%) | 6/53 (11%) | 30/54 (56%) |
| PiB (SUVR), mean ± SEM | 1.11 ± 0.01 | 1.74 ± 0.07 | 1.13 ± 0.01 | 1.92 ± 0.04 |

[Table 2]

**Galectin-3**

Area under the ROC curve (AUC)

| Area under the ROC curve (AUC) | 0.736 |
|---|---|
| Standard Error[a] | 0.0405 |
| 95% Confidence interval[b] | 0.659 to 0.803 |
| 95% Bootstrap CI[c] | 0.649 to 0.809 |
| z statistic | 5.813 |
| Significance level P (Area=0.5) | <0.0001 |

[a] Hanley & McNeil, 1982
[b] Binomial exact
[c] BCa bootstrap confidence interval (1000 iterations; random number seed: 978)

Youden index

| Youden index J | 0.3646 |
|---|---|
| 95% Confidence interval[a] | 0.2096 to 0.4491 |
| Associated criterion | >9.916 |
| 95% Confidence interval[a] | >7.702 to >12.336 |
| Sensitivity | 68.42 |
| Specificity | 68.04 |

**LGALS3BP**

Area under the ROC curve (AUC)

| Area under the ROC curve (AUC) | 0.688 |
|---|---|
| Standard Error[a] | 0.0447 |
| 95% Confidence interval[b] | 0.608 to 0.760 |
| 95% Bootstrap CI[c] | 0.595 to 0.771 |
| z statistic | 4.199 |
| Significance level P (Area=0.5) | <0.0001 |

[a] Hanley & McNeil, 1982
[b] Binomial exact
[c] BCa bootstrap confidence interval (1000 iterations; random number seed: 978)

Youden index

| Youden index J | 0.3149 |
|---|---|
| 95% Confidence interval[a] | 0.1862 to 0.4160 |
| Associated criterion | ≤3.710976 |
| 95% Confidence interval[a] | ≤3.127878665 to ≤7.845184 |
| Sensitivity | 43.86 |
| Specificity | 87.63 |

**EXAMPLE 2. Analysis of the relationship between blood Galectin-3 and blood beta-amyloid levels**

As demonstrated above, the concentration of galectin-3 in the blood showed a positive correlation with beta-amyloid accumulation in the brain, thus the relationship between galectin-3 and the beta-amyloid in the blood were analyzed. As a result, it was found that the blood galectin-3 concentration increases significantly as the concentration of beta-amyloid 1-42 and the ratio 1-42/1-40 in blood decrease (beta-amyloid 1-42, p = 0.0006, r = -0.279; Beta-amyloid 1-42/1-40 concentration ratio, p <0.0001, r = -0.319) (Table 3).

[Table 3]

| | | Gal_3 | A42 | A40 | A4240ratio |
|---|---|---|---|---|---|
| Gal_3 | Correlation coefficient | | -0.279 | 0.032 | -0.319 |
| | Significance Level P | | 0.0006 | 0.7052 | 0.0001 |
| | n | | 146 | 146 | 146 |
| A42 | Correlation coefficient | -0.279 | | 0.371 | 0.661 |
| | Significance Level P | 0.0006 | | <0.0001 | <0.0001 |
| | n | 146 | | 202 | 202 |
| A40 | Correlation coefficient | 0.032 | 0.371 | | -0.389 |
| | Significance Level P | 0.7052 | <0.0001 | | <0.0001 |
| | n | 146 | 202 | | 202 |
| A4240ratio | Correlation coefficient | -0.319 | 0.661 | -0.389 | |
| | Significance Level P | 0.0001 | <0.0001 | <0.0001 | |
| | n | 146 | 202 | 202 | |

Pearson correlation coefficient

**EXAMPLE 3. ROC curve analysis when galectin-3 concentration is added as a variable to the concentration ratio of beta-amyloids in blood**

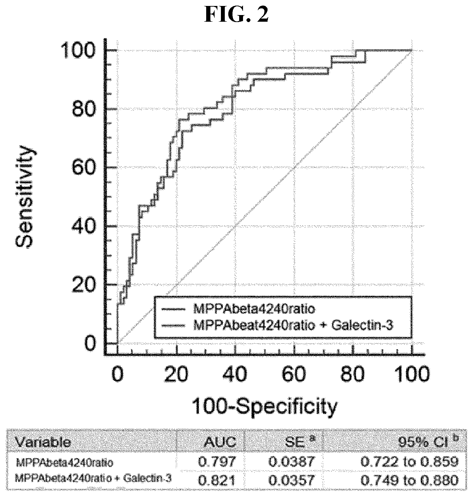

With respect to the concentration ratio of beta-amyloid in blood, when the concentration of galectin-3 was added to the ROC curve as a variable, the degree of improvement of the AUC value was analyzed. The dependent variable was the degree of accumulation of beta-amyloid in the brain (PiB- vs PiB+), and Apolipoprotein E, age, and sex were used as covariates (CV). When ROC curve analysis was performed using only the blood beta-amyloid concentration ratio (42:40 ratio) with the covariate, the AUC value was 0.797, whereas when the concentration variable of galectin-3 was added to this, the AUC value was significantly improved to 0.821 (Fig. 2, Table 4).

This indicates that the effectiveness of the previously reported concentration ratio of beta-amyloid in blood as a biomarker can be increased by addition of the present galectin-3 biomarker for predicting the accumulation of beta-amyloid. It also demonstrates that when the blood concentration of galectin-3 is used as a variable, it can predict the accumulation of beta-amyloid in the brain causing Alzheimer's disease much better than the conventional methods.

[Table 4]

| Variable | AUC | SE [a] | 95% CI [b] |
|---|---|---|---|
| MPPAbeta4240ratio | 0.797 | 0.0387 | 0.722 to 0.859 |
| MPPAbeta4240ratio + Galectin-3 | 0.821 | 0.0357 | 0.749 to 0.880 |

**EXAMPLE 3. ROC curve analysis when adding galectin-3 concentration as a variable compared to the combination of existing biomarkers**

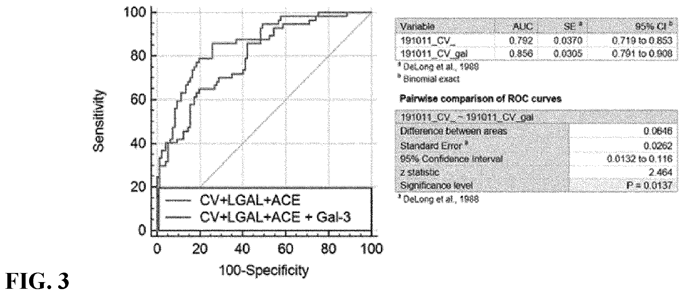

Here it was tested whether the AUC value can be improved when the concentration of galectin-3 is added to the ROC curve as a variable to the existing biomarkers (LGALS3BP, ACE) for predicting blood amyloid brain accumulation. The dependent variable was the degree of accumulation of beta-amyloid in the brain (PiB- vs PiB+), and Apolipoprotein E allele type, age, and sex were used as covariates (CV). When the ROC curve analysis was performed by combining the concentration of LGALS3BP and ACE in the blood with the covariates, the AUC value was 0.792, whereas when the concentration variable of galectin-3 was added, the AUC value was significantly increased to 0.856. (Fig. 3, p=0.0137).

This indicates that the effectiveness of the existing combination markers can be increased by addition of the present galectin-3 biomarker for predicting the accumulation of beta-amyloid. It also demonstrates that when the blood concentration of galectin-3 is used as a variable, it can predict the accumulation of beta-amyloid in the brain causing Alzheimer's disease much better than the conventional methods.

Herein, it was found that the concentration of galectin-3 in the blood is significantly changed as the accumulation of amyloid beta in the brain even in the cognitively normal group and mild cognitive impairment group. Thus it can be effectively used particularly for determining/predicting or detecting the accumulation of cerebral amyloid beta and the early diagnosis of related diseases in the cognitively normal group and mild cognitive impairment group.

Unless defined otherwise, all technical and scientific terms and any acronyms used herein have the same meanings as commonly understood by one of ordinary skill in the art in the field of the invention.

**Claims**

1. A composition for use in detection of the accumulation of amyloid beta in the brain of a subject having a normal cognitive function or mild cognitive impairment, comprising an agent for detecting Galectin-3 as a biomarker in the blood of the subject.

2. The composition of claim 1, wherein the concentration of biomarker Galectin -3 is increased in the blood of the subject having a normal cognitive function and mild cognitive impairment.

3. The composition of claims 1 or 2, wherein the biomarker further comprises at least one of ACE (angiotensin converting enzyme), LGALS3BP, and ApoE.

4. The composition of anyone of claims 1 to 3, wherein the biomarker further comprises beta-amyloid 1-42 or a ratio of 1-42/1-40 in the blood, and the beta-amyloid 1-42 and the ratio of 1-42/1-40 is decreased in the blood of the subject having a normal cognitive function and mild cognitive impairment.

5. The composition of anyone of claims 1 to 4, wherein the brain amyloid beta accumulation is used for early diagnosis of diseases related to brain amyloid beta accumulation, or for determining whether a Pittsburgh compound B (PIB)-positron emission tomography (PET) test is necessary for the subject, wherein the disease related to the brain amyloid beta accumulation disease includes Alzheimer's disease, Parkinson's disease dementia, Lewy body dementia, Huntington's disease dementia, or pre-clinical Alzheimer's disease, Down syndrome, or cognitive impairment.

6. A method of detecting a brain beta amyloid accumulation of a subject in need of thereof, comprising the steps of:

   providing a blood sample from the subject;
   quantifying Galectin-3 biomarker in the blood sample; and
   correlating the quantified result of the biomarker with a brain amyloid beta accumulation in the subject;
   wherein the subject belongs to a cognitively normal group or mild cognitive impairment group.

7. The method of claim 6, wherein the correlation step further comprises determining that the subject has a brain amyloid beta accumulation when the amount of the Galectin-3 biomarker is increased compared to the amount determined in the control group.

8. The method of claims 6 or 7, wherein the biomarker further comprises at least one of LGALS3BP, ACE (Angiotensin Converting Enzyme), or ApoE, and
   wherein the correlation step further comprises determining that the subject has a brain amyloid beta accumulation when the amount of LGALS3BP is decreased, the amount of ACE is decreased in comparison to the amount determined in the control group, or the subject has a ApoE 4 allele.

9. The method of anyone of claims 6 to 8,

   wherein the biomarker further comprises a beta-amyloid 1-42 or a ratio of 1-42/1-40 in blood, and
   wherein the correlation step further comprises determining that the subject has a brain amyloid beta accumulation when the amount of the beta-amyloid 1-42 or the ratio of 1-42/1-40 in the blood is decreased in comparison to those of the biomarkers determined in the control group.

10. The method of claim 9, wherein the blood beta-amyloid is treated with MPP (Mixture of protease and phosphatase).

11. Use of Galectin-3 for detecting a beta-amyloid accumulation in the brain.

# FIG. 1

## FIG. 2

| Variable | AUC | SE [a] | 95% CI [b] |
|---|---|---|---|
| MPPAbeta4240ratio | 0.797 | 0.0387 | 0.722 to 0.859 |
| MPPAbeta4240ratio + Galectin-3 | 0.821 | 0.0357 | 0.749 to 0.880 |

FIG. 3

| Variable | AUC | SE [a] | 95% CI [b] |
|---|---|---|---|
| 191011_CV_ | 0.792 | 0.0370 | 0.719 to 0.853 |
| 191011_CV_gal | 0.856 | 0.0305 | 0.791 to 0.908 |

[a] DeLong et al., 1988
[b] Binomial exact

**Pairwise comparison of ROC curves**

| 191011_CV_ ~ 191011_CV_gal | |
|---|---|
| Difference between areas | 0.0646 |
| Standard Error [a] | 0.0262 |
| 95% Confidence Interval | 0.0132 to 0.116 |
| z statistic | 2.464 |
| Significance level | P = 0.0137 |

[a] DeLong et al., 1988

# FIG. 4

## INTERNATIONAL SEARCH REPORT

| International application No. |
| --- |
| **PCT/KR2019/014027** |

**A. CLASSIFICATION OF SUBJECT MATTER**

*G01N 33/68(2006.01)i*

According to International Patent Classification (IPC) or to both national classification and IPC

**B. FIELDS SEARCHED**

Minimum documentation searched (classification system followed by classification symbols)
G01N 33/68

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: Alzheimer's disease, amyloid beta(β-amyloid), galectin-3, biomarker

**C. DOCUMENTS CONSIDERED TO BE RELEVANT**

| Category* | Citation of document, with indication, where appropriate, of the relevant passages | Relevant to claim No. |
| --- | --- | --- |
| X | WANG, Xuexin et al. Elevated galectin-3 levels in the serum of patients with Alzheimer's disease. American Journal of Alzheimer's Disease & Other Dementias. 2015, vol. 30, no. 8, pages 729-732<br>See abstract, pages 729-730 and Fig. 1. | 1,2,6,7,11 |
| Y | | 3,8 |
| Y | FUH, Jong-ling et al. Serum galectin-3 level is associated with cognitive decline in Alzheimer's disease patients. Alzheimer's Association International Conference. 24 July 2018, page P1171<br>See page P1171. | 3,8 |
| A | VENKATRAMAN, Anand et al. Galectin-3 and incident cognitive impairment in REGARDS, a cohort of blacks and whites. Alzheimer's & Dementia: Translational Research & Clinical Interventions. 26 April 2018, vol. 4, pages 165-172<br>See abstract, pages 165-167. | 1-3,6-8,11 |
| A | GARRANZO-ASENSIO, Maria et al. Identification of prefrontal cortex protein alterations in Alzheimer's disease. Oncotarget. 24 January 2018, vol. 9, no. 13, pages 10847-10867<br>See abstract, pages 10848, 10851-10856. | 1-3,6-8,11 |

☒ Further documents are listed in the continuation of Box C.　　☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 21 FEBRUARY 2020 (21.02.2020) | **21 FEBRUARY 2020 (21.02.2020)** |

| Name and mailing address of the ISA/KR | Authorized officer |
| --- | --- |
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

**INTERNATIONAL SEARCH REPORT**

International application No.

**PCT/KR2019/014027**

C (Continuation). DOCUMENTS CONSIDERED TO BE RELEVANT

| Category* | Citation of document, with indication, where appropriate, of the relevant passages | Relevant to claim No. |
|---|---|---|
| A | KALLO, Gergo et al. Changes in the chemical barrier composition of tears in Alzheimer's disease reveal potential tear diagnostic biomarkers. Plos One. 21 June 2016, vol. 11, no. 6, pages 1-14<br>See pages 2-9. | 1-3,6-8,11 |

Form PCT/ISA/210 (continuation of second sheet) (January 2015)

## INTERNATIONAL SEARCH REPORT

| International application No. |
| --- |
| **PCT/KR2019/014027** |

**Box No. II      Observations where certain claims were found unsearchable (Continuation of item 2 of first sheet)**

This international search report has not been established in respect of certain claims under Article 17(2)(a) for the following reasons:

1. ☐  Claims Nos.:
    because they relate to subject matter not required to be searched by this Authority, namely:

2. ☒  Claims Nos.: **10**
    because they relate to parts of the international application that do not comply with the prescribed requirements to such an extent that no meaningful international search can be carried out, specifically:

    Claim 10 refers to claim 9 which violates the manner of referring to a dependent claim (PCT Rule 6.4(a)), and thus is unclear.

3. ☒  Claims Nos.: **4, 5, 9**
    because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐  As all required additional search fees were timely paid by the applicant, this international search report covers all searchable claims.

2. ☐  As all searchable claims could be searched without effort justifying additional fees, this Authority did not invite payment of additional fees.

3. ☐  As only some of the required additional search fees were timely paid by the applicant, this international search report covers only those claims for which fees were paid, specifically claims Nos.:

4. ☐  No required additional search fees were timely paid by the applicant. Consequently, this international search report is restricted to the invention first mentioned in the claims; it is covered by claims Nos.:

**Remark on Protest**      ☐  The additional search fees were accompanied by the applicant's protest and, where applicable, the payment of a protest fee.

☐  The additional search fees were accompanied by the applicant's protest but the applicable protest fee was not paid within the time limit specified in the invitation.

☐  No protest accompanied the payment of additional search fees.

Form PCT/ISA/210 (continuation of first sheet (2)) (January 2015)

**INTERNATIONAL SEARCH REPORT**
Information on patent family members

| International application No. |
| --- |
| **PCT/KR2019/014027** |

| Patent document cited in search report | Publication date | Patent family member | Publication date |
| --- | --- | --- | --- |

None

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20120041823
- KR 20100049363
- KR 20140042331
- KR 20160129444
- US 481656 A
- KR 1786859

**Non-patent literature cited in the description**

- **SCHEUNER et al.** *Nature Medicine,* 1996, vol. 2, 864-870
- **GALASKO et al.** *Arch Neurol,* 1998, vol. 55 (7), 937-45
- **LI et al.** *Journal of Neurosci ence,* 2011, vol. 31 (35), 12483-12490
- **HAN et al.** *Journal of Alzheimer disease,* 2011, vol. 25 (1), 77-84
- **HEAD, E. ; LOTT, I. T.** Down syndrome and beta-amyloid deposition. *Curr Opin Neurol,* 2004, 17
- **PRIMAVERA et al.** Brain Accumulation of Amyloid-beta in Non-Alzheimer Neurodegeneration. *J Alzheimers Dis,* 1999
- **MASLIAH et al.** beta-amyloid peptides enhance alpha-synuclein accumulation and neuronal deficits in a transgenic mouse model linking Alzheimer's disease and Parkinson's disease. *Proc Natl Acad Sci,* 2001
- **BENSON et al.** *Journal of clinical Psychiatry,* 2006
- **O'BRYANT et al.** *Arch Neurol,* 2008
- **MAWUENYEGA et al.** *Science,* 2010
- **QUERFURTH ; LAFERLA.** *The New England journal of medicine*
- **KOHLER ; MILSTEIN.** *European Journal of Immunology,* 1976, vol. 6, 511-519
- **CLACKSON et al.** *Nature,* 1991, vol. 352, 624-628
- **MARKS et al.** *J. Mol. Biol.,* 1991, vol. 222 (58), 1-597
- **HARLOW, E. ; LANE, D.** Using Antibodies: A Laboratory Manual. Cold Spring Harbor Press, 1999
- **ZOLA, H.** Monoclonal Antibodies: A Manual of Techniques. CRC Press, Inc, 1984
- **COLIGAN.** CURRENT PROTOCOLS IN IMMUNOLOGY. Wiley/Greene, 1991
- **HAN, S.H. et al.** *Neurobiol Aging,* 2019, vol. 73, 21-29
- **REIMAN, E.M. et al.** *Proc Natl Acad Sci U S A,* 2009, vol. 106 (16), 6820-5
- **CHOE, Y.M. et al.** *Neurobiol Aging,* 2014, vol. 35 (7), 1519-25
- **PARK, J.C. et al.** *Brain,* 2019, vol. 142 (3), 771-786